# EUROPÄISCHE PATENTSCHRIFT

(11) **EP 2 012 717 B1**
(45) Veröffentlichungstag und Bekanntmachung des Hinweises auf die Patenterteilung: **08.06.2011**
(21) Anmeldenummer: 07723990.3
(22) Anmeldetag: 04.04.2007
(51) Int. Cl.: A61F 5/01

(54) **KOMPRESSIVE ORTHESE**
COMPRESSIVE ORTHOSIS
ORTHÈSE COMPRESSIVE

(30) Priorität: 26.04.2006 DE 202006006884 U
(43) Veröffentlichungstag der Anmeldung: 14.01.2009
(73) Patentinhaber: Maheshwar, Dayal, 45359 Essen (DE)
(72) Erfinder: Maheshwar, Dayal, 45359 Essen (DE)
(74) Vertreter: Nunnenkamp, Jörg
(86) Internationale Anmeldenummer: PCT/EP2007/003049
(87) Internationale Veröffentlichungsnummer: WO 2007/124841

(56) Entgegenhaltungen:
- EP-A- 0 639 361
- DE-A1- 3 512 997
- DE-A1-102004 036 344
- DE-A1-102005 017 587
- DE-T2- 69 917 456
- DE-U1- 29 808 232
- US-A- 4 832 010
- US-A- 5 036 838
- US-A- 5 896 676
- US-A1- 2005 113 729

## Beschreibung

Die Erfindung betrifft eine kompressive Orthese in Gestalt eines eine menschliche Extremität aufnehmenden Kompressionsstrumpfes, mit wenigstens einem schlauchartigen Basiskörper aus einem elastisch isotropen und nachgebenden Grundmaterial, welches mit durchgängig in Längsrichtung verlaufenden sowie im Wesentlichen äquidistant zueinander angeordneten Einschnürungen ausgebildet ist.

Derartige Orthesen, also orthopädische Einrichtungen, insbesondere zur Behandlung von Gliedmaßen, werden im Allgemeinen dazu eingesetzt, Schwellungen, respektive Ödeme zu behandeln. Zugehörige diagnostische Indikationen solcher Ödeme oder Lymphöderne bedeuten, dass die jeweilige Extremität (beispielsweise ein Bein oder ein Arm) der zu behandelnden Person aufgrund von Flüssigkeitsansammlungen geschwollen ist Bisher hat man solche Schwellungen durch Anlegen eines Kompressionsverbandes eingedämmt und nach und nach beseitigt (medizinisch als Akutphase bezeichnet).

Beim Anlegen der vorgenannten Kompressionsverbände oder Kompressionsbinden ergeben sich jedoch Schwierigkeiten dergestalt, dass die zur Beherrschung der Schwellung einzustellende Vorspannung kaum exakt definiert und eingehalten werden kann. Aus diesem Grund müssen solche Kompressionsverbände zumeist von Fachpersonal angelegt werden. Das ist aufwändig und teuer.

Folgerichtig hat es in der Vergangenheit mit beispielsweise dem Gebrauchsmuster DE 298 08 232 U1 bereits Ansätze gegeben, Schwierigkeiten beim Wickeln zu überwinden. Dazu schlägt das genannte Gebrauchsmuster einen Kompressionsverband zum Stützen des Gewebes im Bereich zwischen Fuß und Knie vor, welcher beispielsweise zur Behandlung venöser und/oder lymphatischer Schwellungen geeignet ist. Der bekannte Kompressionsverband ist mit einem fertigen Fußteil ausgebildet, Nichtsdestotrotz schließt sich an einen oberen Abschluss des Fußteils eine Kompressionsbinde an, die unverändert um die jeweilige Extremität gewickelt werden muss.

Daneben kennt man so genannte Kompressionsstrümpfe oder Kompressionsstrumpfhosen durch die DE 699 17 456 T2. Hier wird eine starke Kompression der aufgenommenen Extremität bzw. der jeweiligen Gliedmaßen so erreicht, dass der schlauchartige Basiskörper aus einem elastischen Material hergestellt ist, typischerweise eine gestrickte Masche von sehr dicht gepackter Textur aufweist. Im Detail sind bei der genannten Veröffentlichung die kompressiven Maschen solche vom Typ eines gestrickten Gitters. Dabei sind aufeinander folgende Reihen von Schussfäden aus elastischem Material vorgesehen, welche derart geknüpft sind, um ein Gitter von Zellen zu formen. Kurz und gut sieht die bekannte Lehre eine bestimmte Machart in Gestalt eines gestrickten Gitters mit einem Schussfaden aus elastischem Material vor. Aufgrund des dadurch aufgebauten Druckes auf die in dem schlauchartigen Basiskörper aufgenommene Extremität ist das Anlegen des bekannten Kompressionsstrumpfes nicht immer einfach, zumal vorliegend überwiegend Elastanfäden zum Einsatz kommen, die sich nicht unbedingt durch eine günstige Gleitfähigkeit über die Haut der aufzunehmenden Extremität auszeichnen.

Ganz abgesehen davon befasst sich die DE 10 2004 036 344 A1 mit einer Strickware zum Stützen und/oder Komprimieren von Körperteilen und/oder zur Kompressionsbehandlung. Diese Strickware weist bereichsweise wenigstens einen elastischen Strickfaden und mindestens einen elastischen Schussfaden auf und ist auf einer Flachstrickmaschine als Schlauch gestrickt hergestellt. Auch in diesem Fall kann die Bedienung nicht restlos überzeugen und sind im Übrigen die Kosten nicht unerheblich.

Im Rahmen der gattungsbildenden Lehre nach der EP 0 639 361 A1 wird mit durchgängigen Längsnähten gearbeitet, welche zur Verbindung von zwei halbzylinderartigen Schalen zu dem schlauchartigen Grundkörper dienen. Ähnlich geht die US 5,896,676 vor. In beiden Fällen werden spezielle Maßnahmen zur Behandlung von Ödemen oder allgemein Schwellungen nicht angesprochen.

Sofern die US 2005/0113729 A1 eine kompressive Orthese beschreibt, ist der bekannte Aufbau mit einzelnen Kompressionsstreifen in Querrichtung der aufgenommenen menschlichen Extremität kompliziert. Das gilt nicht nur für das Anlegen, sondern auch das Spannen der einzelnen Kompressionsstreifen.

Schließlich ist durch die DE 10 2005 017 587 A1 eine Bandage bekannt geworden, die durch regelmäßig beabstandete Druckbereiche gekennzeichnet ist. Dabei verfügt wenigstens ein Druckbereich über eine Fläche größer oder gleich 200 mm².

Der Erfindung liegt das technische Problem zugrunde, eine kompressive Orthese der eingangs beschriebenen Ausgestaltung so weiter zu entwickeln, dass die Bedienung und insbesondere das Anlegen gegenüber sonstigen Ausgestaltungen deutlich erleichtert sind und eine insgesamt kostengünstige Ausführungsform zur Verfügung gestellt wird.

Zur Lösung dieser technischen Problemstellung ist eine gattungsgemäße kompressive Orthese im Rahmen der Erfindung dadurch gekennzeichnet, dass die Einschnürungen einen jeweiligen Abstand von ca. 5 mm bis 15 mm aufweisen, so dass der Basiskörper in Richtung seines Querschnittes eine geringe Elastizität aufweist, wohingegen in seiner Längsrichtung eine große Elastizität gegeben ist, wobei das Verhältnis der Elastizitäten wenigstens 1 : 3 beträgt.

Im Rahmen der Erfindung kommt also ausdrücklich keine Strickware oder überhaupt ein textiles Flächengebilde zum Einsatz, wie es in den Schriften DE 699 17 456 T2 und DE 10 2004 036 344 A1 beschrieben wird. Ähnlich geht das Gebrauchsmuster DE 298 08 232 U1 vor. Vielmehr greift die Erfindung auf ein flächiges elastisch nachgebendes Grundmaterial zurück, welches über isotrope Elastizitätseigenschaften verfügt, also im Idealfall in sämtlichen drei Raumrichtungen gleiche oder doch zumindest ähnliche Elastizitätseigenschaften aufweist.

Dieses hinsichtlich seiner Elastizitätseigenschaften isotrope Grundmaterial wird nun erfindungsgemäß noch dadurch modifiziert, dass in Längsrichtung der im schlauchartigen Basiskörper aufgenommenen jeweiligen Extremität (in der Regel handelt es sich hierbei um ein Bein oder einen Arm) Einschnürungen verlaufen. Denn diese Einschnürungen sorgen dafür, dass das elastisch isotrope Grundmaterial hinsichtlich seiner Dehnungseigenschaften derart
(hieran schließen sich die ursprünglichen Beschreibungsseiten 4 bis 12 in unveränderter Reihenfolge an) modifiziert wird, dass der hieraus hergestellte schlauchartige Basiskörper über einen nahezu formstabilen Querschnitt entlang seiner gesamten Länge verfügt. D. h., die in Längsrichtung verlaufenden Einschnürungen sorgen dafür, dass die Elastizität des Grundmaterials bei dem daraus hergestellten schlauchartigen Basiskörper so verändert worden ist, dass relative Formstabilität in Querschnittsrichtung des schlauchartigen Basiskörpers erreicht wird bzw. nur noch geringe Dehnungen zugelassen werden, wohingegen die Elastizität des Grundmaterials in Längsrichtung des schlauchartigen Basiskörpers praktisch nicht beeinträchtigt wird.

Dadurch entfaltet die erfindungsgemäße kompressive Orthese die gewünschte Kompressionswirkung, weil Schwellungen durch die Formstabilität des schlauchartigen Basiskörpers in Querschnittsrichtung eingedämmt und beseitigt werden. Gleichzeitig sorgt die besondere Elastizität in Längsrichtung des schlauchartigen Basiskörpers dafür, dass die Bewegungsfreiheit der Extremität praktisch nicht beeinträchtigt wird und das Anlegen der erfindungsgemäßen Orthese einfach und problemlos vonstatten geht. D. h., die Orthese lässt sich problemlos als beispielsweise Strumpf oder Strumpfhose anziehen, wobei Beugebewegungen des Ellenbogens oder Kniegelenkes durch die große Elastizität in Längsrichtung des schlauchartigen Basiskörpers praktisch kaum beeinträchtigt werden. Hierin sind die wesentlichen Vorteile zu sehen.

Im Detail hat es sich bewährt, wenn das Grundmaterial schaumartig und/oder schwammartig mit zugehörigen geschlossenen und/oder offenen Zellen ausgeführt ist. Denn solche schaumartigen und/oder schwammartigen Werkstoffe verfügen automatisch über die geforderte elastische Isotropie. Dabei kommen überwiegend Schaumstoffe zum Einsatz, also schaumartige und mit geschlossenen Zellen ausgerüstete sowie aufgeblähte Kunststoffe. Meistens wird ein thermoplastischer Kunststoff mit geschlossenen Mikroporen von ca. 0,1 mm Durchmesser eingesetzt, welcher eine geringe Feuchtigkeitsaufnahme hat. Bevorzugt wird ein Schaum aus Polyethylen oder auch ein vernetzter Polyester-Kunststoffschaum verwendet, wie z. B. Polyurethan. Der Porengehalt beträgt ca. 60 bis 90 Vol.-% des gesamten Schaumstoffvolumens.

Darüber hinaus hat es sich als günstig erwiesen, wenn das Grundmaterial ein- und/oder beidseitig eine hautfreundliche Abdeckung aufweist. Diese Abdeckung kann ganz oder teilweise aus einem Vliesmaterial auf Basis von Kunststofffasern hergestellt werden. Dabei wird man üblicherweise auf so genannte Hüllvliesstoffe oder medizinische Vliesstoffe zurückgreifen, also beispielsweise Polyestervliesstoffe oder auch Spinnvliesstoffe, welche über eine hohe Querdehnung verfügen. Aufgrund dieser Eigenschaft sind die fraglichen Vliesstoffe in der Lage, gewünschte Körperbewegungen und damit die erforderliche Elastizität des schlauchartigen Basiskörpers in seiner Längsrichtung zur Verfügung zu stellen. Meistens kommt als Grundmaterial für die eingesetzten Synthesefasern bzw. Kunststofffasern zur Herstellung des Vliesstoffes Polyester zum Einsatz, aber auch Polyethylen oder auch Polypropylen.

Darüber hinaus liegt es im Rahmen der Erfindung, die ein- oder beidseitige Abdeckung für das Grundmaterial aus Baumwolle, folglich einer Naturfaser, herzustellen. Dabei mag ein Flächengebilde aus Baumwolle, beispielsweise ein Baumwollgewebe, ein Baumwollgewirke oder auch ein Baumwollvlies zum Einsatz kommen. Insbesondere an der Oberfläche des Grundmaterials, welche der zu umschließenden Extremität zugewandt ist, wird man auf Baumwolle zurückgreifen, um die erforderliche Atmungsaktivität der Orthese insgesamt zu erhalten bzw. überhaupt erst zur Verfügung zu stellen. Dabei begünstigt die beschriebene Innenabdeckung auf Baumwollbasis darüber hinaus weitere oder flankierende Behandlungsmethoden, beispielsweise das Auftragen von Cremes, Ölen usw.. Neben dieser Innenabdeckung auf Baumwollbasis empfiehlt die Erfindung eine optionale Außenabdeckung aus einem vorzugsweise gleitfähigen Flächengebilde. Hier hat sich Satin als günstig erwiesen, also ein Gewebe, welches mit reiner Atlasbindung hergestellt wird. Dadurch ist das zugehörige Flächengebilde bzw. die Außenabdeckung sehr anschmiegsam und fein.

Meistens wird man hier so arbeiten, dass die glänzende Seite des Satins bzw. des Satingewebes bei der Außenabdeckung nach außen weist, während die matte Seite dem Grundmaterial zugewandt ist. Bei der Herstellung des Satins kann auf endlose Fasern aus beispielsweise Viskose oder synthetischen Materialien zurückgegriffen werden. Außerdem lassen sich selbstverständlich vorzugsweise auch Baumwollfasern für die Herstellung des Satins einsetzen, die aufgrund ihrer hautsympathischen Eigenschaften häufig verwandt werden.

Jedenfalls ermöglicht die beschriebene gleitfähige Außenabdeckung, dass die kompressive Orthese bei Bedarf mit einem zusätzlichen Stützstrumpf oder auch einem ergänzenden Kompressionsstrumpf kombiniert werden kann. Denn infolge der besonderen Gleitfähigkeit der Außenabdeckung bzw. des an dieser Stelle vorhandenen Flächengebildes auf Satinbasis lässt sich ein solcher Strumpf problemlos über die angelegte kompressive Orthese ziehen bzw. gleitet auf der Außenabdeckung problemlos. Vergleichbares gilt für den Fall, dass die erfindungsgemäße Orthese mit einer zusätzlichen und von außen aufgebrachten Bandage kombiniert werden soll.

Ferner liegt es auch im Rahmen der Erfindung, als Außenabdeckung auf ein feinmaschiges und vorzugsweise luftdurchlässiges Zweizuggestrick oder allgemein ein vorzugsweise Schlauch-Rund-Gestrick oder eine vergleichbare Strickware zurückzugreifen. Diese Strickware bzw. das Gestrick setzt sich aus jeweils elastischen Strickfäden und elastischen Schussfäden zusammen. Dabei kann der Strickfaden bzw. Grundfaden grundsätzlich auch über eine schwächere Titrierung als der Schussfaden verfügen. So mag man einen Titer des Schussfadens von in etwa 100 dtex beobachten, während der Titer des Grundfadens ca. 30 dtex beträgt. Im Übrigen hat es sich bewährt, wenn die Elastizität des Strickfadens und des Schussfadens im Bereich von ca. 1 % bis ca. 400% liegt. D. h., die fraglichen Fäden können in ihrer Länge im Bereich von 1% bis 400% gedehnt werden. - Grundsätzlich kann als Außenabdeckung auch auf ein elastisches Gewebe aus jeweils elastischen Kett- und Schussfäden zurückgegriffen werden. Die Elastizität der fraglichen Fäden kann in einem vergleichbaren Bereich wie zuvor beschrieben liegen, das heißt zwischen 1 % bis 400% angesiedelt sein.

Dabei sind die durchgängig in Längsrichtung der Orthese verlaufenden Einschnürungen jeweils so ausgebildet, dass sie sowohl die Innenabdeckung als auch die Außenabdeckung und selbstverständlich das dazwischen befindliche Grundmaterial durchdringen. Die Einschnürungen sind im Wesentlichen äquidistant zueinander angeordnet, d. h. sie weisen einen mehr oder minder gleichen Abstand bei ihrem Verlauf in Längsrichtung zueinander auf. Um die Einschnürungen im Detail herzustellen, greift die Erfindung in der Regel auf Längsnähte zurück, die üblicherweise gleiche Abstände zugehöriger Einstiche aufweisen. Die Längsnähte können grundsätzlich als Zickzacknähte, Stretchnähte oder dergleichen ausgeführt werden. In jedem Fall verfügt das den schlauchartigen Basiskörper darstellende Grundmaterial zwischen den Einschnürungen über Längsrippen und werden gegebenenfalls zusätzlich noch Querrippen gebildet. Die Längsrippen gewährleisten die erforderliche Elastizität in Längsrichtung des schlauchartigen Basiskörpers, wohingegen die Querrippen eine unveränderte Beweglichkeit im Bereich der Gelenke oder Muskeln erlauben.

Schließlich können mehrere schlauchartige Basiskörperteile miteinander gekoppelt sein, beispielsweise ein Basiskörperteil für den Unterarm und ein Basiskörperteil für den Oberarm oder ein Basiskörperteil für den Unterschenkel und ein Basiskörperteil für den Oberschenkel. Dabei werden die miteinander gekoppelten Basiskörperteile meistens so miteinander verbunden, dass eine Aussparung im Bereich eines Gelenkes der jeweils im Innern aufgenommenen Extremität vorgesehen ist. Meistens ist die Aussparung so angeordnet, dass sie sich in einem zum Knickbereich des Gelenkes entgegengesetzten Bereich findet.

Zusammenfassend wird eine kompressive Orthese zur Verfügung gestellt, die sich insbesondere zur Behandlung von Ödemen, also Schwellungen, eignet. Diesen wird durch die geringe Elastizität bzw. Formstabilität des schlauchartigen Basiskörpers in Querschnittsrichtung begegnet, wobei in Längsrichtung nach wie vor eine große Elastizität gegeben ist und somit die Bewegungsfreiheit und das Anlegen nicht behindert werden. Gleichzeitig sorgt die Beschichtung mit dem hautfreundlichen Vliesmaterial für einen gesteigerten Tragekomfort, wobei auch die erforderlichen Hygienevorschriften durch den Einsatz von Kunststoffmaterialien für den Basiskörper eingehalten werden. Hierin sind die wesentlichen Vorteile zu sehen.

Im Folgenden wird die Erfindung anhand einer lediglich ein Ausführungsbeispiel darstellenden Zeichnung näher erläutert; es zeigen:
- Fig. 1 und 2: Die erfindungsgemäße kompressive Orthese, einerseits als Beinstrumpf (Fig. 1) und andererseits als Armstrumpf (Fig. 2),
- Fig. 3: einen Schnitt durch den schlauchartigen Basiskörper und
- Fig. 4: eine Detailansicht.

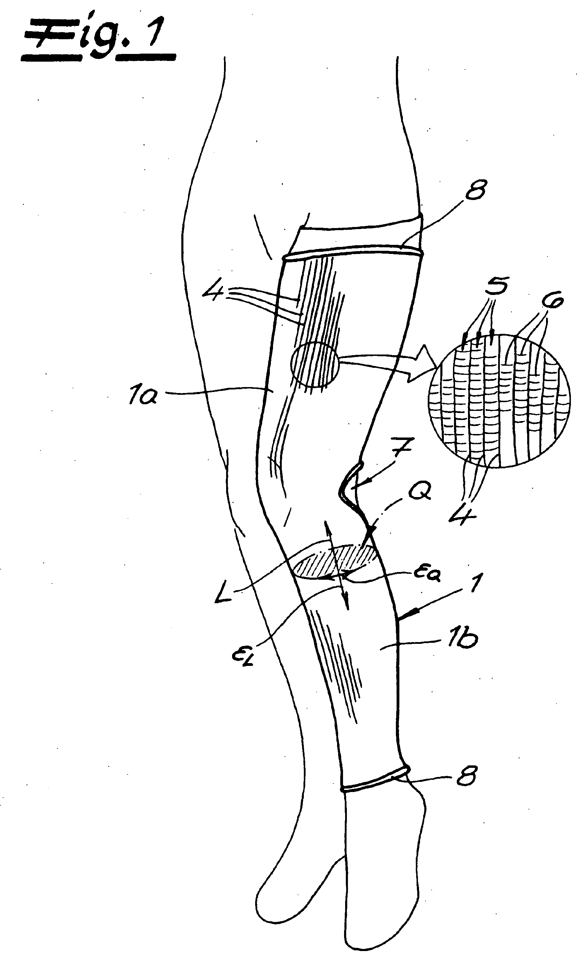

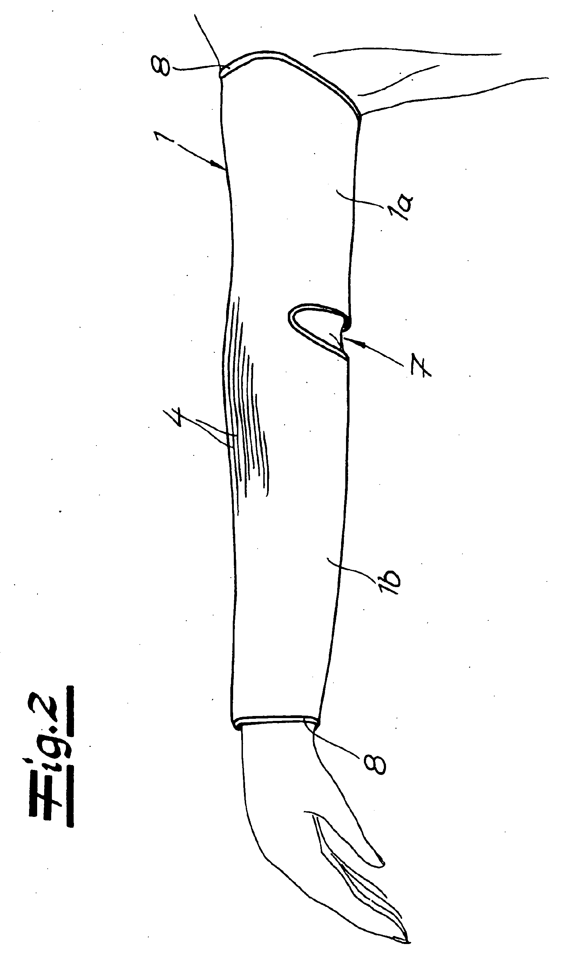

In den Figuren ist eine kompressive Orthese dargestellt, die im Rahmen des Ausführungsbeispiels und nicht einschränkend als Kompressionsstrumpf ausgebildet ist. Der Kompressionsstrumpf nimmt in seinem Innern eine menschliche Extremität auf, im Rahmen der Fig. 1 ein Bein und in der Fig. 2 einen Arm. Folgerichtig handelt es sich bei dem Kompressionsstrumpf nach Fig. 1 um einen Beinstrumpf, während der Kompressionsstrumpf nach Fig. 2 als Armstrumpf ausgeführt ist.

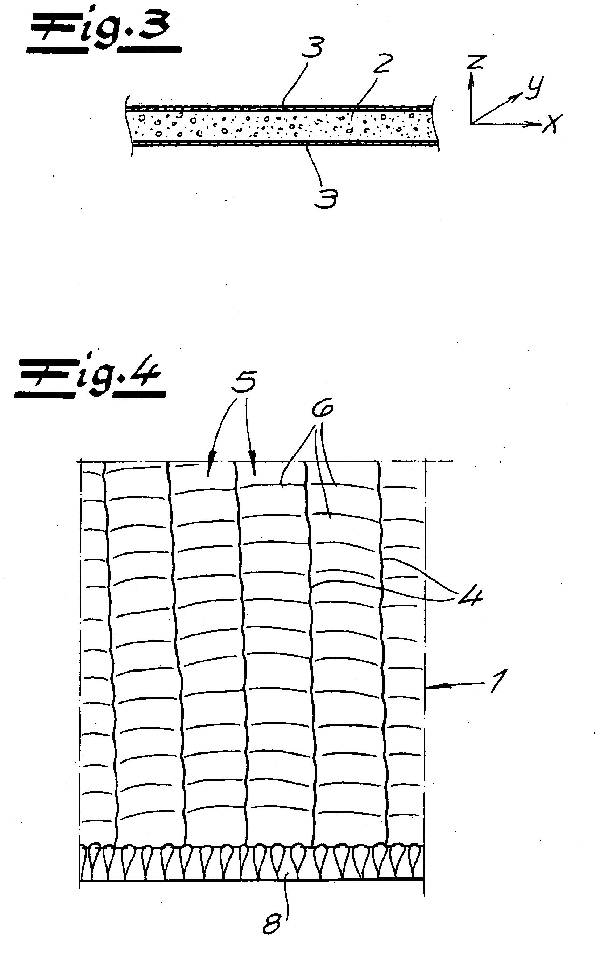

Die kompressive Orthese bzw. der Kompressionsstrumpf verfügt über wenigstens einen schlauchartigen Basiskörper 1 aus einem elastisch nachgebenden Grundmaterial 2, welches man am besten anhand der Schnittdarstellung in der Fig. 3 erkennt. Das Grundmaterial 2 ist vorliegend elastisch isotrop ausgeführt, verfügt also in sämtlichen drei angedeuteten Raumrichtungen X, Y und Z über eine gleiche oder nahezu gleiche Elastizität. Das erreicht die Erfindung dadurch, dass es sich bei dem Grundmaterial 2 im Rahmen der Darstellung um einen Schaumstoff handelt, welcher mit Mikroporen im Bereich kleiner als 0,5 mm ausgerüstet ist und beispielsweise aus Polyethylen hergestellt wird.

Das Grundmaterial 2 ist im Ausführungsbeispiel beidseitig mit einer hautfreundlichen Abdeckung 3 ausgerüstet, bei welcher es sich um eine Abdeckung aus einem Vliesmaterial, insbesondere einem Spinnvlies aus Polyester und/oder Polyethylen, handelt. Die Verbindung zwischen der jeweiligen Abdeckung 3 und dem Grundmaterial 2 wird dadurch hergestellt, dass das Grundmaterial 2 über durchgängige und in Längsrichtung des schlauchartigen Basiskörpers 1 verlaufende Einschnürungen 4 verfügt, die man insbesondere in der Fig. 4 erkennt. Diese Einschnürungen 4 sind äquidistant, d. h. gleich beabstandet, zueinander angeordnet und vorliegend als Längsnähte 4 ausgebildet. Da die Längsnähte bzw. Einschnürungen 4 die beiden Abdeckungen 3 und das dazwischen befindliche Grundmaterial 2 durchdringen, werden mehrere Effekte erreicht.

In diesem Zusammenhang hat es sich als besonders günstig erwiesen, wenn die Längsnähte bzw. Einschnürungen 4 einen jeweiligen Abstand von ca. 5 mm bis 15 mm, vorzugsweise ca. 10 mm aufweisen. Denn dadurch werden die zu behandelnden Schwellungen besonders effektiv behandelt. Außerdem ist die Gesamtdicke des Grundmaterials 2 inklusive der Abdeckung 3 bzw. der Abdeckungen 3 zu ca. 5 mm bis 15 mm bemessen, liegt vorzugsweise im Bereich von ca. 10 mm. Das gewährleistet einen hohen Tragekomfort.

Dadurch, dass die Längsnähte bzw. Einschnürungen 4 die beiden Abdeckungen 3 und das dazwischen befindliche Grundmaterial 2 durchdringen, werden zum einen die beiden Abdeckungen 3 fest mit dem Grundmaterial 2 verbunden. Zum anderen sorgen die Abdeckungen 3 dafür, dass das aus Schaumstoff bestehende Grundmaterial 2 überhaupt mit den Einschnürungen 4 bzw. zugehörigen Längsnähten 4 ausgerüstet werden kann und bei diesem Vorgang nicht zerreißt oder zerfranst. Ferner gewährleisten die Abdeckungen 3 einen Verschleißschutz des im Innern aufgenommenen Grundmaterials 2 und sorgen schließlich die Einschnürungen 4 dafür, dass der schlauchartige Basiskörper 1 in Richtung seines Querschnittes Q eine geringe Elastizität aufweist, wohingegen in seiner Längsrichtung L eine große Elastizität bzw. Dehnbarkeit gegeben ist.

Tatsächlich wird man an dieser Stelle ein Verhältnis der jeweiligen Elastizitäten ε_{Q} für den Querschnitt Q und ε_{L} für die Längsrichtung L von 1 zu 3 bis 5 oder noch mehr erwarten. Das wird in der Fig. 1 durch zugehörige Dehnungsdoppelpfeile ausgedrückt.

Man erkennt einerseits den Querschnitt bzw. eine Querschnittsfläche Q mit ihrem zugehörigen Umfang, der größtenteils formstabil aufgrund der durchgängigen Einschnürungen 4 in Längsrichtung L des Basiskörpers 1 gestaltet ist, wohingegen in der Längsrichtung L große Elastizitäten beobachtet werden. Das drücken die unterschiedlich langen Dehnungsdoppelpfeile ε_{Q} und ε_{L} aus, die das zuvor angegebene Verhältnis von 1 zu 3 oder mehr widerspiegeln.

Dadurch übt der Basiskörper 1 in Richtung des Querschnittes Q den erforderlichen Druck aus, um die zu behandelnden Ödeme respektive Schwellungen in der umschlossenen Extremität einwandfrei zurückdrängen zu können. Gleichzeitig ist ein besonderer Tragekomfort gewährleistet, weil die Bewegungsfreiheit eines zugehörigen Beines in Längsrichtung L praktisch nicht gestört wird und auch das Anlegen des Kompressionsstrumpfes einfach gelingt.

Durch die in Längsrichtung L verlaufenden Einschnürungen 4 bzw. Längsnähte bilden sich zwischen den Einschnürungen 4 jeweils Längsrippen 5, die für die erforderliche Elastizität in Längsrichtung L sorgen. Darüber hinaus erkennt man ansatzweise Querrippen 6, die darauf zurückzuführen sind, dass die Einschnürungen 4 bzw. Längsnähte 4 über Einstiche gleichen Abstandes verfügen und diese Einstiche die Querrippen 6 definieren. Die Querrippen 6 stellen sich also als Folge der Längsnähte 4 ein und erhöhen den Tragekomfort insofern, als Bewegungen in Querrichtung Q bzw. Muskelkontraktionen ebenso zugelassen werden wie Gelenkbewegungen.

Zu diesem Zweck verfügt der schlauchartige Basiskörper 1 zusätzlich noch über eine Aussparung 7 im Bereich eines Gelenkes. Diese Aussparung 7 ist meistens in einem dem Knickbereich des zugehörigen Gelenkes entgegengesetzten Bereich angeordnet und verbindet im Rahmen der Darstellung zwei miteinander gekoppelte Basiskörperteile 1 a, 1b. Jedenfalls sorgt die Aussparung 7 dafür, dass Gelenkbewegungen der in dem schlauchartigen Basiskörper 1 bzw. den mehreren schlauchartigen Basiskörperteilen 1 a, 1b aufgenommenen Extremitäten zugelassen und nicht behindert werden. - Eine umlaufende Saumnaht 8 stellt schließlich sicher, dass der Basiskörper 1; 1a, 1 b im Randbereich nicht ausfranst und insbesondere die Abdeckungen 3 einwandfrei mit dem Grundmaterial 2 die gewünschte Verbindung eingehen, und zwar auch im Randbereich.

Selbstverständlich liegt es im Rahmen der Erfindung, die beidseitig des Grundmaterials 2 vorhandenen Abdeckungen 3 aus unterschiedlichen Materialien herzustellen. Dabei hat sich nicht nur Vliesmaterial auf Basis von Kunststofffasern für die Abdeckung 3 als günstig erwiesen, sondern werden vorteilhaft auch Flächengebilde auf Baumwollbasis eingesetzt. Ebenso kann die Abdeckung 3 auch aus Satin oder einer Strickware hergestellt sein. Im Rahmen einer vorteilhaften Variante greift die nach außen weisende Abdeckung 3, die Außenabdeckung, auf ein Flächengebilde aus Satin zurück. Dagegen ist die innenseitige Abdeckung 3 bzw. Innenabdeckung auf Baumwollbasis hergestellt. Hierdurch kann über die beschriebene kompressive Orthese problemlos ein zusätzlicher Kompressionsstrumpf gezogen werden, eine außenseitige Bandage angebracht werden oder dergleichen. Bei einer anderen Variante kann der fragliche Kompressionsstrumpf gleichsam in die beschriebene Orthese integriert werden.

Dann ist die äußere Abdeckung 3 bzw. die Außenabdeckung als Strickware aus elastischen Strickfäden und Schussfäden aufgebaut, die insgesamt ein Schlauchgestrick bilden, welches vorteilhaft auf einer Flachstrickmaschine hergestellt ist. Die Elastizität der Strickfäden und der Schussfäden kann im Bereich vom ca. 1% bis 400% liegen. Außerdem verfügen die fraglichen Fäden vorteilhaft über eine Feinheit von ca. 20 bis ca. 3.000 dtex. In diesem Fall und auch ansonsten wird man die innere Abdeckung 3 bzw. Innenabdeckung auf Basis eines Bauwollflächengebildes herstellen. Dabei sind - wie bereits beschriebenen - die beiden Abdeckungen 3 selbstverständlich nicht zwingend, sondern können wahlweise entweder nur eine oder beide Abdeckungen 3 mit dem Grundmaterial 2 über die Längsnähte 4 verbunden werden.

## Patentansprüche

1. Kompressive Orthese in Gestalt eines eine menschliche Extremität aufnehmenden Kompressionsstrumpfes, mit wenigstens einem schlauchartigen Basiskörper (1; 1a, 1b) aus einem elastisch isotropen und nachgebenden Grundmaterial (2), welches mit durchgängig in Längsrichtung (L) verlaufenden sowie im Wesentlichen äquidistant zueinander angeordneten Einschnürungen (4) ausgebildet ist,
**dadurch gekennzeichnet, dass**
- die Einschnürungen (4) einen jeweiligen Abstand von ca. 5 mm bis 15 mm aufweisen, so dass
- der Basiskörper (4) in Richtung seines Querschnittes (Q) eine geringe Elastizität (ε_{Q}) aufweist, wohingegen in seiner Längsrichtung (L) eine große Elastizität (ε_{L}) gegeben ist, wobei das Verhältnis der Elastizitäten (ε_{Q}: ε_{L}) wenigstens 1 : 3 beträgt.

2. Orthese nach Anspruch 1, **dadurch gekennzeichnet, dass** das Grundmaterial (2) schaumartig und/oder schwammartig mit zugehörigen geschlossen und/oder offenen Zellen ausgeführt ist.

3. Orthese nach Anspruch 1 oder 2, **dadurch gekennzeichnet, dass** das Grundmaterial (2) ein- und/oder beidseitig eine Abdeckung (3) aufweist.

4. Orthese nach Anspruch 3, **dadurch gekennzeichnet, dass** die Abdeckung (3) ganz oder teilweise aus einem Vliesmaterial hergestellt ist.

5. Orthese nach einem der Ansprüche 1 bis 4, **dadurch gekennzeichnet, dass** die Einschnürungen (4) als Längsnähte (4) mit vorzugsweise gleichem Abstand zugehöriger Einstiche ausgebildet sind.

6. Orthese nach einem der Ansprüche 1 bis 5, **dadurch gekennzeichnet, dass** das Grundmaterial (2) zwischen den Einschnürungen (4) verlaufende Längsrippen (5) und gegebenenfalls Querrippen (6) aufweist.

7. Orthese nach einem der Ansprüche 1 bis 6, **dadurch gekennzeichnet, dass** mehrere schlauchartige Basiskörperteile (1a, 1b) miteinander gekoppelt sind.

8. Orthese nach einem der Ansprüche 1 bis 7, **dadurch gekennzeichnet, dass** der Basiskörper (1; 1a, 1b) eine Aussparung (7) im Bereich eines Gelenkes der jeweils aufgenommenen Extremität aufweist.

9. Orthese nach einem der Ansprüche 1 bis 8, **dadurch gekennzeichnet, dass** die Aussparung (7) in einem einem Knickbereich des Gelenkes entgegengesetzten Bereich angeordnet ist.

## Claims

1. Compressive orthosis in the form of a compression hose holding a human extremity, with at least one tube-like base body (1; 1 a, 1 b) of an elastically isotropic and yielding base material (2), which is formed with constrictions (4) running continuously in the longitudinal direction (L) and arranged substantially equidistantly from each other,
**characterized in that**
- the constrictions (4) have a respective distance of approx. 5 mm to 15 mm, so that
- the base body (4) has a low elasticity (e_{Q}) in the direction of its cross-section (Q), whereas in its longitudinal direction (L) there is a high lasticity (e_{L}), wherein the ratio of the elasticities (e_{Q} : e_{L}) is at least 1 : 3.

2. Orthosis according to Claim 1, **characterized in that** the base material (2) is embodied so as to be foam-like and/or sponge-like with associated closed and/or open cells.

3. Orthosis according to Claim 1 or 2, **characterized in that** the base material (2) has a covering (3) on one and/or both sides.

4. Orthosis according to Claim 3, **characterized in that** the covering (3) is produced in whole or in part from a non-woven material.

5. Orthosis according to one of Claims 1 to 4, **characterized in that** the constrictions (4) are formed as longitudinal seams (4) with preferably an equal distance of associated grooves.

6. Orthosis according to one of Claims 1 to 5, **characterized in that** the base material (2) has longitudinal ribs (5), running between the constrictions (4), and if applicable transverse ribs (6).

7. Orthosis according to one of Claims 1 to 6, **characterized in that** several tube-like base body parts (1a, 1b) are coupled with each other.

8. Orthosis according to one of Claims 1 to 7, **characterized in that** the base body (1; 1a, 1b) has an opening (7) in the region of a joint of the extremity which is respectively held.

9. Orthosis according to one of Claims 1 to 8, **characterized in that** the opening (7) is arranged in a region opposed to a bending region of the joint.

## Revendications

1. Orthèse compressive ayant la forme d'un bas de contention prenant une extrémité humaine, comprenant au moins un corps de base tubulaire (1 ; 1 a, 1 b) dans un matériau de base (2) élastique isotrope et extensible, lequel est formé de constrictions (4) constantes dans le sens longitudinal (L) et essentiellement équidistantes entre elles,
- **caractérisé en ce que** les constrictions (4) présentent un écart respectif d'environ 5 mm à 15 mm, de façon à ce que
- le corps de base (4) présente une faible élasticité (ε_{Q}) en direction de sa section transversale (Q), mais par contre une grande élasticité (ε_{L}) est donnée dans sa direction longitudinale (L), sachant que le rapport des élasticités (ε_{Q:} ε_{L}) est au moins de 1 : 3.

2. Orthèse selon la revendication 1, **caractérisée en ce que** le matériau de base (2) est semblable à de la mousse et/ou de l'éponge avec des cellules correspondantes ouvertes et/ou fermées.

3. Orthèse selon la revendication 1 ou 2, **caractérisée en ce que** le matériau de base (2) présente un recouvrement (3) sur un côté et/ou les deux côtés.

4. Orthèse selon la revendication 3, **caractérisée en ce que** le recouvrement (3) est fabriqué totalement ou partiellement dans un matériau non-tissé.

5. Orthèse selon l'une des revendications 1 à 4, **caractérisée en ce que** les constrictions (4) sont formées comme des coutures longitudinales (4) avec des piqûres correspondantes de préférence avec la même distance.

6. Orthèse selon l'une des revendications 1 à 5, **caractérisée en ce que** le matériau de base (2) présente des rainures longitudinales (5) et le cas échéant des rainures transversales (6) entre les constrictions (4).

7. Orthèse selon l'une des revendications 1 à 5, **caractérisée en ce que** plusieurs parties de corps de base (1a, 1b) tubulaires sont couplées entre elles.

8. Orthèse selon l'une des revendications 1 à 7, **caractérisée en ce que** le corps de base (1 ; 1 a, 1 b) présente un évidement (7) au niveau d'une articulation de l'extrémité respective prise.

9. Orthèse selon l'une des revendications 1 à 8, **caractérisée en ce que** l'évidement (7) est placé dans une zone opposée à une zone de pli de l'articulation.
